(19) **Europäisches Patentamt / European Patent Office / Office européen des brevets**

(11) **EP 4 328 216 B1**

(12) **EUROPEAN PATENT SPECIFICATION**

(45) Date of publication and mention
of the grant of the patent:
**08.07.2026 Bulletin 2026/28**

(21) Application number: **22899138.6**

(22) Date of filing: **29.11.2022**

(51) International Patent Classification (IPC):
**C07C 59/01** (2006.01)    **C07C 51/48** (2006.01)
**C07C 51/43** (2006.01)    **C12P 7/52** (2006.01)
**C12N 9/88** (2006.01)    **C07C 51/02** (2006.01)

(52) Cooperative Patent Classification (CPC):
**C07C 59/01; C07C 51/02; C07C 51/43;
C07C 51/48; C12P 7/42;** C07B 2200/13

(86) International application number:
**PCT/KR2022/019065**

(87) International publication number:
**WO 2023/096456 (01.06.2023 Gazette 2023/22)**

(54) **CRYSTAL OF 3-HYDROXYPROPIONIC ACID SALT, METHOD FOR PRODUCING SAME, AND PROCESS FOR RECOVERING 3-HYDROXYPROPIONIC ACID**

KRISTALL EINES 3-HYDROXYPROPIONSÄURESALZES, VERFAHREN ZU SEINER HERSTELLUNG UND VERFAHREN ZUR RÜCKGEWINNUNG VON 3-HYDROXYPROPIONSÄURE

CRISTAL DE SEL D'ACIDE 3-HYDROXYPROPIONIQUE, SON PROCÉDÉ DE PRODUCTION ET PROCÉDÉ DE RÉCUPÉRATION D'ACIDE 3-HYDROXYPROPIONIQUE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **29.11.2021 KR 20210167406**
           **29.11.2021 KR 20210167409**

(43) Date of publication of application:
**28.02.2024 Bulletin 2024/09**

(73) Proprietor: LG Chem, Ltd.
**Seoul 07336 (KR)**

(72) Inventors:
• **AHN, Sangbum**
  **Daejeon 34122 (KR)**
• **JUNG, Woochul**
  **Daejeon 34122 (KR)**
• **HAN, Sang Won**
  **Daejeon 34122 (KR)**
• **JEONG, Yongbok**
  **Daejeon 34122 (KR)**
• **WOO, Seung A**
  **Daejeon 34122 (KR)**
• **KIM, Jimyeong**
  **Daejeon 34122 (KR)**

(74) Representative: **Goddar, Heinz J. et al
Boehmert & Boehmert
Anwaltspartnerschaft mbB
Pettenkoferstrasse 22
80336 München (DE)**

(56) References cited:
**WO-A1-2015/166098**      **US-B2- 8 883 464**

• **URBANUS J ET AL:** "Intensified crystallization in complex media: Heuristics for crystallization of platform chemicals", CHEMICAL ENGINEERING SCIENCE, OXFORD, GB, vol. 77, 13 February 2012 (2012-02-13), pages 18 - 25, XP028500286, ISSN: 0009-2509, [retrieved on 20120224], DOI: 10.1016/J.CES.2012.02.019

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- J. URBANUS; C.P.M. ROELANDS; D. VERDOES; J.H. TER HORST;: "Intensified crystallization in complex media: Heuristics for crystallization of platform chemicals", CHEMICAL ENGINEERING SCIENCE, OXFORD, GB, vol. 77, 13 February 2012 (2012-02-13), GB , pages 18 - 25, XP028500286, ISSN: 0009-2509, DOI: 10.1016/j.ces.2012.02.019
- MCDONALD MATTHEW A, HOSSEIN SALAMI, PATRICK R. HARRIS, COLTON E. LAGERMAN, XIAOCHUAN YANG, ANDREAS S. BOMMARIUS, MARTHA A. GROVER,: "Reactive crystallization: a review", REACTION CHEMISTRY & ENGINEERING, THE ROYAL SOCIETY OF CHEMISTRY, vol. 6, no. 3, 1 March 2021 (2021-03-01), pages 357 - 574, XP055951360, ISSN: 2058-9883, DOI: 10.1039/D0RE00272K
- RAMACHANDRAN SUMITRA ,FONTANILLE PIERRE, PANDEY ASHOK, LARROCHE CHRISTIAN: "Gluconic acid: Properties, applications and microbial production", FOOD TECHNOLOGY AND BIOTECHNOLOGY, SVEUCILISTE U ZAGREBU * PREHRAMBENO-BIOTEHNOLOSKI FAKULTET, CROATIA, vol. 44, no. 2, 1 April 2006 (2006-04-01), Croatia , pages 185 - 195, XP002670029, ISSN: 1330-9862

**Description**

## FIELD OF THE INVENTION

The present invention relates to a 3-hydroxypropionate crystal, a preparation method of the same, and a process of recovering 3-hydroxypropionic acid.

## BACKGROUND OF THE INVENTION

3-Hydroxypropionic acid (3HP) is a platform compound that can be converted to various chemicals such as acrylic acid, methyl acrylate, and acrylamide. Since being identified as one of the Top 12 value-added bio-chemicals by the US Department of Energy (DOE) in 2004, it has been actively researched in academic and industrial world.

The production of 3-hydroxypropionic acid is mainly carried out by two methods, chemical and biological, but in the case of chemical method, it has been pointed out that the initial material is expensive and that it is non-environmental because toxic substances are generated during the production process, and thus, environmentally friendly bioprocesses have been attracting attention.

When organic acids are produced by microbial fermentation, other by-products are produced in addition to organic acids such as 3-hydroxypropionic acid in the process of fermenting microorganisms, and thus, a process of extracting and separating organic acids from the fermentation liquor is needed.

The recovery of 3-hydroxypropionic acid from a fermentation broth that involves separation followed by crystallisation is known from CHEMICAL ENGINEERING SCIENCE, vol. 77, 13 February 2012.

Therefore, there is a need to develop a method that can provide 3-hydroxypropionic acid in a high purity and a high yield even through a biological method.

## SUMMARY OF THE INVENTION

### TECHNICAL PROBLEM

It is an object of the present invention to provide a 3-hydroxypropionate crystal that not only contains very low amounts of impurities, but also is easy to purify and has excellent thermal stability and shape stability.

It is another object of the present invention to provide a method for preparing a high-purity 3-hydroxypropionate crystal that is easy to purify and has excellent thermal and shape stability.

It is yet another object of the present invention to provide a process of recovering 3-hydroxypropionic acid from the 3-hydroxypropionate crystal provided above.

### TECHNICAL SOLUTION

Provided herein is a 3-hydroxypropionate crystal which: comprises a calcium salt of 3-hydroxypropionic acid or a hydrate thereof, or a magnesium salt of 3-hydroxypropionic acid or a hydrate thereof, and comprises a first peak at a diffraction angle ($2\theta \pm 0.2°$) of 11.0 to 13.0 in an X-ray diffraction spectrum (XRD).

Also provided is a method for preparing a 3-hydroxypropionate crystal, comprising: forming a concentrate containing 100 g/L or more of 3-hydroxypropionic acid in the presence of $Ca(OH)_2$, $Mg(OH)_2$ or a mixture thereof; and contacting the concentrate with a nonsolvent to form a 3-hydroxypropionate crystal.

Further provided is a process of recovering 3-hydroxypropionic acid, comprising separating the 3-hydroxypropionate crystal obtained in the method for preparing the 3-hydroxypropionate crystal, and converting the separated crystal to 3-hydroxypropionic acid.

Further embodiments are disclosed in the dependent claims.

Now, a 3-hydroxypropionate crystal, a method for preparing a 3-hydroxypropionate crystal and a process of recovering 3-hydroxypropionic acid according to embodiments of the present invention will be described in more detail.

It should be understood that, unless steps included in a preparation method described herein are specified as being sequential or consecutive or otherwise stated, one step and another step included in the preparation method should not be construed as being limited to an order described herein. Therefore, it should be understood that an order of steps included in a preparation method can be changed within the range of understanding of those skilled in the art, and that, in this case, the incidental changes obvious to those skilled in the art fall within the scope of the present invention.

According to one embodiment of the present invention, there can be provided a 3-hydroxypropionate crystal which: comprises a calcium salt of 3-hydroxypropionic acid or a hydrate thereof; or a magnesium salt of 3-hydroxypropionic acid or a hydrate thereof, and comprises a first peak at a diffraction angle ($2\theta \pm 0.2°$) of 11.0 to 13.0 in an X-ray diffraction spectrum (XRD).

The present inventors have found through experiments that 3-hydroxypropionate crystals formed by contacting a 3-hydroxypropionic acid concentrate formed under predetermined conditions with a nonsolvent has the above-mentioned specific peaks in the X-ray diffraction spectrum (XRD), and that not only such 3-hydroxypropionate crystals contain very low amounts of impurities and are easy to purify compared to amorphous substances, but also they are excellent in shape stability and thermal stability in a crystalline state, making it possible to efficiently mass-produce 3-hydroxypropionic acid having high purity, and completed the invention.

Specifically, when a nonsolvent is brought into contact with the formed 3-hydroxypropionic acid concentrate in the presence of acalcium or magnesium salt, a calcium or magnesium salt of 3-hydroxypropionic acid is produced, and as the concentration of the produced calcium or magnesium salt of 3-hydroxypropionic acid increases, microcrystals are formed, and solid-liquid phase separation occurs. After that, as crystallization proceeds, the calcium or magnesium salt of 3-hydroxypropionic acid grows into solid crystals (3-hydroxypropionate crystal), and the resultant 3-hydroxypropionate crystal contain impurities at a very low content while being separated from liquid impurities such as glycerol and 1,3-propanediol. At this time, the nonsolvent serves to promote crystallization of the calcium or magnesium salt of 3-hydroxypropionic acid, so that the solid-liquid separation ability is increased and 3-hydroxypropionate crystal having higher purity can be produced.

The crystalline state of such a 3-hydroxypropionate crystal can be confirmed through peaks and the like in an X-ray diffraction (XRD) graph.

As mentioned above, the 3-hydroxypropionate crystal may exhibit a first peak at a diffraction angle ($2\theta \pm 0.2°$) of 11.0 to 13.0, or 11.3 to 12.7, or 11.8 to 12.50 in an X-ray diffraction spectrum (XRD) during X-ray diffraction (XRD) analysis.

Such peaks show different results from the X-ray diffraction (XRD) analysis results for magnesium hydroxide ($Mg(OH)_2$) or magnesium sulfate ($Mg(SO_4)$). As a result of the X-ray diffraction (XRD) analysis, it can be confirmed that when a specific peak appears in the above-mentioned $2\theta$ value range, the 3-hydroxypropionate crystal of the above embodiment is formed.

Specifically, in the case of calcium or magnesium salt of 3-hydroxypropionic acid, it has a different morphology from the existing crystal phases such as inorganic calcium and magnesium salt, which are difficult to analyze with the naked eye or SECM due to its morphological characteristics. However, since the X-ray diffraction (XRD) analysis value differs depending on the binding structure of the crystal-forming metal (e.g., calcium or magnesium) and 3-hydroxypropionic acid, the 3-hydroxypropionate crystal of the above embodiment can be confirmed by analyzing each inherent peak that appears by measuring the X-ray diffraction spectrum (XRD) of the 3-hydroxypropionate crystal.

Further, as described below, the molecular size (length) of metal-3HP in the 3-hydroxypropionate crystals can be confirmed through the $2\theta$ values for each peak position, and the intermolecular distance between each molecule can also be confirmed.

At this time, the first peak described above and the peak generated other than the second to fourth peaks to be described later overlap or are adjacent with the peaks of the inorganic salt form at the distance between a metal element and an adjacent element, or at a distance between metal and metal, so as to be different from the peaks of the 3-hydroxypropionate crystals of the above embodiment, which forms organic bonds.

The first peak appearing at the diffraction angle ($2\theta \pm 0.2°$) of 11.0 to 13.0 in the X-ray diffraction spectrum (XRD) represents the length (size) of the salt molecule formed by the combination of two molecules of metal and 3-hydroxypropionic acid, and the length (size) of a salt molecule composed of a metal and two 3-hydroxypropionic acids can be found through Bragg's law of Equation 1 below.

[Equation 1]

$$n*\lambda = 2d*\sin\theta$$

In Equation 1, n is 1, $\lambda$ is 1.54 Å as a Cu-intrinsic constant, and d means the distance (length) from the bonding surface or the distance (length) of (between) molecules.

At this time, since the d value calculated at the first peak by Equation 1 is about 7.0 to 7.5, and the length of one molecule of 3-hydroxypropionic acid is about 4.3 Å, it can be inferred therefrom that due to the bonding structure of two molecules of 3-hydroxypropionic acid and a metal (e.g., calcium or magnesium), 3-HP is disposed at a chemically stable energy level into the center of the metal to form a molecular structure in a bent form.

Further, during X-ray diffraction (XRD) analysis for the 3-hydroxypropionate crystal of the above embodiment, 1 or more, 3 or more, 5 or more, 7 or more, 9 or more peaks may appear in the $2\theta$ value range of 10 to 30°.

More specifically, the 3-hydroxypropionate crystal may include a second peak at a diffraction angle ($2\theta \pm 0.2°$) of 19.0 to 21.0 in an X-ray diffraction spectrum (XRD), or may include a third peak at a diffraction angle ($2\theta \pm 0.2°$) of 22.0 to 23.80 in an X-ray diffraction spectrum (XRD), or may include a fourth peak at a diffraction angle ($2\theta \pm 0.2°$) of 24.0 to 25.00 in an X-ray diffraction spectrum (XRD), during X-ray diffraction (XRD) analysis.

At this time, the length of one molecule of 3-hydroxypropionic acid can be inferred through the second peak appearing at the diffraction angle ($2\theta \pm 0.2°$) of 19.0 to 21.0 in the X-ray diffraction spectrum (XRD).

Specifically, the d value calculated for the second peak by Equation 1 is approximately the half of the d value calculated for the first peak, thereby being able to infer the length (size) of one 3-hydroxypropionic acid molecule, which is half the length (size) of a salt molecule composed of a metal and two 3-hydroxypropionic acids.

Further, through the third peak at the diffraction angle ($2\theta \pm 0.2°$) of 22.0 to 23.80 and the fourth peak at the diffraction angle ($2\theta \pm 0.2°$) of 24.0 to 25.00 in the X-ray diffraction spectrum (XRD), the distance between salt molecules composed of a metal and two 3-hydroxypropionic acids, that is, the distance between salt molecules can be inferred.

Meanwhile, the incident angle ($\theta$) means the point at which a first differential value (slope of the tangent line, dy/dx) is 0 on a graph where the horizontal axis (x-axis) in the x-y plane is a value of two times the incident angle ($2\theta$) of the incident X-ray, and the vertical axis (y-axis) in the x-y plane is a diffraction intensity, wherein as the value ($2\theta$) of two times the angle of incidence of the incident X-ray, which is the horizontal axis (x-axis), increases in the positive direction, the first differential value (slope of the tangent line, dy/dx) of two times ($2\theta$) the incident angle of X-rays, which is the horizontal axis (x-axis), with respect to the diffraction intensity, which is the vertical axis (y-axis), changes from a positive value to a negative value.

The grain domain size of 3-hydroxypropionate calculated as the full width at half maximum at the first peak appearing at the diffraction angle ($2\theta \pm 0.2°$) of 11.0 to 13.0 in the X-ray diffraction spectrum (XRD) may be 30 nm or more, 40 nm or more, or 50 nm or more, and may be 100 nm or less, 90 nm or less, or 80 nm or less.

When drawing a straight line parallel to the abscissa passing through a point that is half the ordinate value of the peak height in a distribution curve with one or more peaks, and the curves intersect at two points, the full width at half maximum (FWHM) refers to the distance between these two points, and the full width at half maximum (FWHM) of the peak obtained from the results obtained during X-ray diffraction (XRD) analysis is related to the crystallite size.

At this time, when calculating the crystal size, it is generally calculated assuming that the peak shape is a bell shape with left and right symmetry. The actual peak shape is not left and right symmetrical, and peak broadening may appear due to the effects of strain, etc., and the full width at half maximum (FWHM) may be calculated using a Lorenzian method, ignoring such peak broadening.

As described above, when a nonsolvent is brought into contact with the formed 3-hydroxypropionic acid concentrate in the presence of a calcium or magnesium salt, acalcium or magnesium salt of 3-hydroxypropionic acid is produced. While the concentration of the calcium or magnesium salt of 3-hydroxypropionic acid produced is increasing, a microcrystal is formed. Then, as crystallization progresses, it grows into solid crystals (3-hydroxypropionate crystals).

That is, the calcium or magnesium metal salt of 3-hydroxypropionic acid forms a microcrystal, the microcrystal grows to a micro size that can be measured optically by the ratio of nonsolvent, drying conditions, etc., and crystallization progresses to form 3-hydroxypropionate crystals.

At this time, the grain domain size of 3-hydroxypropionate calculated as the full width at half maximum in the first peak may increase or decrease depending on conditions, but the gran domain size of 30 to 100 nm facilitates the removal of impurities and is excellent in workability.

If the gran domain size is too large, micro-sized crystals are easily produced, but it is difficult to remove impurities, and if the gran domain size is too small, the removal of impurities is facilitated, but microcrystals grow and the crystallization time that can be recovered may become longer.

Through the microcrystal size at the first peak appearing at the diffraction angle ($2\theta \pm 0.2°$) of 11.0 to 13.0 in the X-ray diffraction spectrum (XRD), the size of microcrystals [the size of the seed] of the organic acid salt crystals produced by the binding of a calcium or magnesium metal and two molecules of 3HP can be determined.

Meanwhile, the gran domain size of the 3-hydroxypropionate can be calculated using the Scherer equation of Equation 2 below.

[Equation 2]

$$\tau = (K^* \lambda) / \beta\cos\theta$$

in Equation 2, K is 0.9 as a shape factor, $\lambda$ is 1.54 Å as a Cu intrinsic constant, $\beta$ is the full width at half maximum (FWHM, radians) of the peak, and $\tau$ means the gran domain size (nm) of 3-hydroxypropionate.

Meanwhile, the 3-hydroxypropionate crystal may include acalcium or magnesium salt of 3-hydroxypropionic acid or a hydrate thereof, and may be in the form of the following structural formula 1 or structural formula 2.

In the following structural formula 1 and structural formula 2, Cation may be $Mg^{2+}$ or $Ca^{2+}$.

3HP below means 3-hydroxypropionic acid that binds to a cation,

n is the number of 3HP that binds to the cation and may be an integer of 1 or more, and more specifically 2.

In the following structural formula 2, m is the number of water molecules bonded to Cation(3HP)n in the hydrate, and may be an integer of 1 or more, and more specifically, 2.

[Structural Formula 1] $\quad$ Cation$(3HP)_n$

[Structural Formula 2] $\quad$ Cation$(3HP)_n \cdot mH_2O$

The particle size distribution $D_{50}$ of the 3-hydroxypropionate crystal may be 1 $\mu$m or more, 5 $\mu$m or more, 8 $\mu$m or more, or 10 $\mu$m or more, and may be 200 $\mu$m or less, 180 $\mu$m or less, or 160 $\mu$m or less.

The particle size distribution $D_{10}$ of the 3-hydroxypropionate crystal may be 2 $\mu$m or more and 90 $\mu$m or less, 3 $\mu$m or more and 80 $\mu$m or less, or 4 $\mu$m or more and 78 $\mu$m or less.

The particle size distribution $D_{90}$ of the 3-hydroxypropionate crystal may be 10 $\mu$m or more and 250 $\mu$m or less, 15 $\mu$m or more and 210 $\mu$m or less, or 20 $\mu$m or more and 205 $\mu$m or less.

The particle size distributions $D_{50}$, $D_{10}$ and $D_{90}$ mean particle sizes at which cumulative volumes of particles reach 50%, 10% and 90%, respectively, in the particle size distribution curve of the particles, wherein the $D_{50}$, $D_{10}$ and $D_{90}$ can be measured using, for example, a laser diffraction method. The laser diffraction method can generally measure particle sizes ranging from a submicron range to several millimeters, and can obtain results with high reproducibility and high resolvability.

Meanwhile, the $(D_{90}\text{-}D_{10})/D_{50}$ of the 3-hydroxypropionate crystal may be 0.70 or more and 3.00 or less, or 0.75 or more and 2.90 or less.

If the particle size distributions $D_{50}$, $D_{10}$ and $D_{90}$ of the 3-hydroxypropionate crystal is too large, impurities that must be removed during crystallization may be contained in the crystals, which results in a reduction in purification efficiency. If the particle size distributions are too small, the liquid permeability during filtration of the crystals may be lowered.

If the volume average particle size, number average particle size, and volume average particle size of the 3-hydroxypropionate crystal are too large, impurities that must be removed during crystallization may be contained in the crystals, which results in a reduction in purification efficiency. If they are too small, the liquid permeability during filtration of the crystals may be lowered.

Meanwhile, the 3-hydroxypropionate crystal having crystal characteristics defined as the peak at the diffraction angle ($2\theta \pm 0.2°$) in the X-ray diffraction spectrum (XRD) contains a very low content of impurities. More specifically, the 3-hydroxypropionate crystal may have a content of residual components including glycerol, 1,3-propanediol, and acetate of 100 ppmw or less or 50 ppmw or less.

The purity of 3-hydroxypropionate contained in the 3-hydroxypropionate crystal may be calculated as a percentage (%) of the mass of the compound having the structural formula 1 and/or structural formula 2 relative to the mass of the total crystal recovered. The purity of 3-hydroxypropionate contained in the 3-hydroxypropionate crystal may be 70% or more, 80% or more, 90% or more, 95% or more, 98% or more, 99% or more, 99.8% or more, or 99.9% or more.

Meanwhile, the 3-hydroxypropionate crystal can measure the moisture content contained in the crystals by the Karl Fischer method, and the moisture content contained in the 3-hydroxypropionate crystal may be 200 ppm or more and 5000 ppm or less, 250 ppm or more and 4800 ppm or less, 300 ppm or more and 4600 ppm or less, or 350 ppm or more and 4400 ppm or less.

At this time, the moisture contained in the 3-hydroxypropionate crystal means the attached moisture contained between the crystals, rather than the crystal moisture (for example, $Ca(3HP)_2 \cdot 2H_2O$). Further, if the moisture content contained in the 3-hydroxypropionate crystal is too high, it may be recovered in the form of a slurry rather than a crystalline solid, or impurities may be contained in the water, which may cause a problem of a decrease in purity improvement.

Meanwhile, as 3-hydroxypropionic acid is prepared through a process such as fermenting a bacterium strain having 3-hydroxypropionic acid-producing ability, the 3-hydroxypropionate crystal may contain a radioactive carbon isotope ($^{14}C$).

The radioactive carbon isotope ($^{14}C$) contains about 1 atom per $10^{12}$ carbon atoms in earth's atmosphere and has a half-life of about 5700 years, and carbon stocks can become abundant in the upper atmosphere due to nuclear reactions in which cosmic rays and normal nitrogen ($^{14}N$) participate. Meanwhile, in fossil fuels, radioactive isotopes have decayed long ago, so that the $^{14}C$ ratio may be substantially zero. When bio-derived raw materials are used as the 3-hydroxypropionic acid raw material, or fossil fuels are used together with it, the content of radioactive carbon isotopes (pMC; percent modern carbon) and the content of bio-carbon contained in 3-hydroxypropionic acid can be measured according to the standard of ASTM D6866-21.

After carbon atoms contained in the compound to be measured is made into graphite form or carbon dioxide gas form, the content can be measured, for example, by a mass spectrometer, or can be measured according to a liquid scintillation analysis method. At this time, the two isotopes can be separated using an accelerator for separating $^{14}C$ ions from $^{12}C$ ions together with the mass spectrometer, and the content and the content ratio can be measured by a mass spectrometer.

The 3-hydroxypropionate crystal has a radiocarbon isotope content of 20 pMC (percent modern carbon) or more, 50 pMC or more, 90 pMC or more, or 100 pMC or more, and a biocarbon content of 20 wt.% or more, 50 wt.% or more, 80 wt.%, 90 wt.%, or 95 wt.%, as measured according to the standard of ASTM D6866-21.

The radiocarbon isotope ratio (pMC) means the ratio of the radiocarbon isotope ($^{14}$C) contained in the 3-hydroxypropionate crystals to the radiocarbon isotope ($^{14}$C) of the modern standard reference material, and it can be greater than 100% because the nuclear test program of the 1950s is still in effect and not extinguished.

Further, the content of biocarbon means the content of biocarbon relative to the total carbon content contained in the 3-hydroxypropionate crystal. As this value is larger, it may correspond to an environmentally friendly compound.

Meanwhile, if the radiocarbon isotope content (pMC) and biocarbon content of the 3-hydroxypropionate crystal are too low, the environmental friendliness is reduced, which may not be seen as a bio-derived material.

Meanwhile, according to another embodiment of the invention, there can be provided a method for preparing a 3-hydroxypropionate crystal, comprising: forming a concentrate containing 100 g/L or more of 3-hydroxypropionic acid in the presence of $Ca(OH)_2$, $Mg(OH)_2$ or a mixture thereof; and contacting the concentrate with a nonsolvent to form a 3-hydroxypropionate crystal.

The present inventors confirmed that when a nonsolvent is brought into contact with the formed 3-hydroxypropionic acid concentrate in the presence of a calcium or magnesium salt, pure 3-hydroxypropionate crystals can be formed by controlling the crystal formation rate as compared to crystals formed at a high rate due to overconcentration, and then the crystal size can be increased.

Such 3-hydroxypropionate crystals contain very low amounts of impurities inside the crystals, and have excellent filterability due to uniform crystal formation, so that not only it is easy to purify, but it is also excellent in shape stability and thermal stability in the crystalline state, thereby being able to efficiently mass-produce 3-hydroxypropionic acid having high purity.

Specifically, when a nonsolvent is brought into contact with the formed 3-hydroxypropionic acid concentrate in the presence of acalcium or magnesium salt, a calcium or magnesium salt of 3-hydroxypropionic acid is produced, and while the concentration of the produced calcium or magnesium salt of 3-hydroxypropionic acid is increasing, microcrystals are formed, and solid-liquid phase separation occurs. After that, as crystallization proceeds, the calcium or magnesium salt of 3-hydroxypropionic acid grows into solid crystals (3-hydroxypropionate crystal), and the resultant 3-hydroxypropionate crystal contains impurities at a very low content while being separated from liquid impurities such as glycerol and 1,3-propanediol. At this time, the nonsolvent serves to promote crystallization of the calcium or magnesium salt of 3-hydroxypropionic acid, so that the solid-liquid separation ability is increased and 3-hydroxypropionate crystal having higher purity can be produced.

The concentrate containing 3-hydroxypropionic acid may contain 3-hydroxypropionic acid at a concentration of 100 g/L or more, 150 g/L or more, 200 g/L or more, 250 g/L or more, 300 g/L or more, 350 g/L or more, 400 g/L or more, 450 g/L or more, or 500 g/L or more, and may contain 3-hydroxypropionic acid at a concentration of 1000 g/L or less, 950 g/L or less, 900 g/L or less, 850 g/L or less, or 800 g/L or less.

Whether or not the crystal of 3-hydroxypropionic acid is formed appears to be affected by the presence of a cacium or magnesium salt, the concentration of 3-hydroxypropionic acid in the concentrate, and the like.

Further, when the concentration of the crystal of 3-hydroxypropionic acid in the concentrate is higher than the water solubility of the crystal of 3-hydroxypropionic acid, the crystal of 3-hydroxypropionic acid can be more easily produced.

For example, the water solubility of $Ca(3HP)_2$, which is a crystal of 3-hydroxypropionic acid, is 450 g/L at room temperature, and thus, when the concentration of 3-hydroxypropionic acid in the concentrate exceeds 450 g/L, the formation of $Ca(3HP)_2$ crystal can be promoted. Further, the water solubility of $Mg(3HP)_2$, which is a crystal of 3-hydroxypropionic acid, is 250 g/L at room temperature, and thus, when the concentration of 3-hydroxypropionic acid in the concentrate exceeds 250 g/L, the formation of $Mg(3HP)_2$ crystal can be promoted.

The 3-hydroxypropionate crystal can be formed from a concentrate satisfying the above-mentioned concentration even while containing the calcium or magnesium salt. The calcium or magnesium salt may be selected without limitation within the purpose range for forming 3-hydroxypropionate crystal. It may include one or more cations selected from the group consisting of $Mg^{2+}$ and $Ca^{2+}$. Further, the calcium or magnesium salt may be $Ca(OH)_2$, $Mg(OH)_2$, or a mixture thereof.

The calcium or magnesium salt is added and remains in the process of producing the 3-hydroxypropionic acid fermentation liquid, or it can be added in the process of forming 3-hydroxypropionate crystal in the concentrate containing 100 g/L or more of 3-hydroxypropionic acid. Further, the concentration of the calcium or magnesium salt may be 10% to 100%, or 30% to 90% of the 3-hydroxypropionic acid concentration, and for example, it can be present in the concentrate at a concentration of 10 to 900 g/L, 50 to 800 g/L, 100 to 700 g/L, or 200 to 600 g/L.

As mentioned above, 3-hydroxypropionate crystals having a predetermined crystal characteristic may be formed in the process of contacting the concentrate containing 100 g/L or more of 3-hydroxypropionic acid with a nonsolvent.

The 3-hydroxypropionate crystal includes a calcium or magnesium salt of 3-hydroxypropionic acid or a hydrate thereof, and has a first peak at a diffraction angle ($2\theta \pm 0.2°$) of 11.0 to 13.0, 11.3 to 12.7, or 11.8 to 12.50 in the X-ray diffraction spectrum (XRD) during X-ray diffraction (XRD) analysis.

Further, during X-ray diffraction (XRD) analysis for the 3-hydroxypropionate crystal, 1 or more, 3 or more, 5 or more, 7 or more, 9 or more peaks may appear in the $2\theta$ value range of 10 to 30°.

During the X-ray diffraction (XRD) analysis, the 3-hydroxypropionate crystal may comprise a second peak at a diffraction angle ($2\theta \pm 0.2°$) of 19.0 to 21.0 in an X-ray diffraction spectrum (XRD), or comprise a third peak at a diffraction angle ($2\theta \pm 0.2°$) of 22.0 to 23.80 in an X-ray diffraction spectrum (XRD), or comprise a fourth peak at a diffraction angle ($2\theta \pm 0.2°$) of 24.0 to 25.00 in an X-ray diffraction spectrum (XRD).

The molecular size (length) of metal-3HP in the 3-hydroxypropionate crystals can be confirmed through the $2\theta$ values for each peak position, and the intermolecular distance between each molecule can also be confirmed.

Specifically, the length (size) of the salt molecule composed of the metal and two 3-hydroxypropionic acids can be known through the first peak, the length of one molecule of 3-hydroxypropionic acid can be known through the second peak, and the distance between salt molecules composed of the metal and two 3-hydroxypropionic acids can be known through the third peak and the fourth peak.

The details regarding each peak are as described above.

Meanwhile, the gran domain size of 3-hydroxypropionate calculated as the full width at half maximum at the first peak appearing at the diffraction angle ($2\theta \pm 0.2°$) of 11.0 to 13.0 in the X-ray diffraction spectrum (XRD) may be 30 nm or more, 40 nm or more, or 50 nm or more, and 100 nm or less, 90 nm or less, or 80 nm or less.

The particle size distribution $D_{50}$ of the 3-hydroxypropionate crystal may be 1 $\mu$m or more, 5 $\mu$m or more, 8 $\mu$m or more, or 10 $\mu$m or more, or may be 200 $\mu$m or less, 180 $\mu$m or less, or 160 $\mu$m or less.

Further, the particle size distribution $D_{10}$ of the 3-hydroxypropionate crystal may be 2 $\mu$m or more and 90 $\mu$m or less, 3 $\mu$m or more and 80 $\mu$m of less, or 4 $\mu$m or more and 78 $\mu$m or less.

Further, the particle size distribution $D_{90}$ of the 3-hydroxypropionate crystal may be 10 $\mu$m or more and 250 $\mu$m or less, 15 $\mu$m or more and 210 $\mu$m or less, or 20 $\mu$m or more and 205 $\mu$m or less.

The ($D_{90}$-$D_{10}$)/$D_{50}$ of the 3-hydroxypropionate crystal may be 0.70 or more and 3.00 or less, or 0.75 or more and 2.90 or less.

In the process of forming the 3-hydroxypropionate crystal, the volume ratio between the concentrate containing 100 g/L or more of 3-hydroxypropionic acid and the nonsolvent can be determined by considering the concentration of 3-hydroxypropionic acid or the volume of the concentrate and nonsolvent. For example, the concentrate and the nonsolvent may be used in a volume ratio of 1:0.5 to 1:20, or 1:0.5 to 1:10, or 1:0.8 to 1:8, or 1:1 to 1:5.

If the volume of the nonsolvent is too small compared to the concentrate, the concentration at which crystals are formed is high, and the rate of crystal formation is increased, so that pure crystal particles are not formed slowly and irregular crystal particles are rapidly formed. Further, the solid-liquid separation ability is low, which may make it difficult to separate liquid impurities from the calcium or magnesium salt of organic acid to be purified.

In addition, if the volume of the nonsolvent is too large compared to the concentrate, the formed crystals may be melted and dissolved in some cases due to the solubility of the nonsolvent, and the time in the crystal filtration process after crystal formation increases, and the amount of waste liquid increases, which may be uneconomical.

The nonsolvent may include an alcohol-based nonsolvent, a ketone-based nonsolvent, a nitrile-based nonsolvent, or a mixture of two or more thereof, and more specifically, at least one alcohol-based nonsolvent may be used.

The ketone-based nonsolvent may include one or more selected from the group consisting of acetone, methyl ethyl ketone, cyclohexanone, diethyl ketone, acetophenone, methyl isobutyl ketone, methyl isoamyl ketone, isophorone and di-(isobutyl)ketone.

The alcohol-based nonsolvent may include one or more selected from the group consisting of methanol, ethanol, allyl alcohol, 1-propanol, 2-propanol, 1-butanol, 2-butanol, isobutanol, benzyl alcohol, cyclohexanol, diacetone alcohol, ethylene glycol monoethyl ether, diethylene glycol monomethyl ether, diethylene glycol monoethyl ether, ethylene glycol monobutyl ether, diethylene glycol monobutyl ether, 2-methoxyethanol and 1-decanol.

The nitrile-based nonsolvent may include one or more selected from the group consisting of acetonitrile, propionitrile, butyronitrile, valeronitrile, caprylonitrile, heptanenitrile, cyclopentane carbonitrile, cyclohexane carbonitrile, 2-fluorobenzonitrile, 4-fluorobenzonitrile, difluorobenzonitrile, trifluorobenzonitrile, phenylacetonitrile, 2-fluorophenylacetonitrile, and 4-fluorophenylacetonitrile.

In addition, the step of forming 3-hydroxypropionate crystal may further comprise stirring the concentrate.

The stirring step can be carried out at a temperature of 0 to 70 degrees Celsius, 0 to 60 degrees Celsius, 0 to 50 degrees Celsius, 0 to 40 degrees Celsius, 0 to 35 degrees Celsius, 0 to 30 degrees Celsius, 10 to 70 degrees Celsius, 10 to 60 degrees Celsius, 10 to 50 degrees Celsius, 10 to 40 degrees Celsius, 10 to 35 degrees Celsius, 10 to 30 degrees Celsius, 15 to 70 degrees Celsius, 15 to 60 degrees Celsius, 15 to 50 degrees Celsius, 15 to 40 degrees Celsius, 15 to 35 degrees Celsius, 15 to 30 degrees Celsius, 20 to 70 degrees Celsius, 20 to 60 degrees Celsius, 20 to 50 degrees Celsius, 20 to 40 degrees Celsius, 20 to 35 degrees Celsius, or 20 to 30 degrees Celsius (e.g., room temperature), and/or under conditions of 100 to 2000rpm, 100 to 1500rpm, 100 to 1000rpm, 100 to 500rpm, 100 to 400rpm, or 200 to 400rpm (e.g., about 300rpm).

The step of contacting the concentrate with a nonsolvent to form a 3-hydroxypropionate crystal may be carried out at a temperature of 0°C or more, 15°C or more, 30°C or more, 35°C or more, 40°C or more, 45°C or more, 50°C or more, or 55°C or more, and may be carried out at a temperature of 100°C or less, 90°C or less, 80°C or less, 75°C or less, 70°C or less, 65°C or less, or 0°C to 100°C.

At this time, the temperature may be adjusted to the above range by adding a nonsolvent at the above-mentioned temperature to the concentrate, or by heating or cooling in a state in which the concentrate and the nonsolvent are mixed.

Meanwhile, the step of forming a concentrate containing 100 g/L or more of 3-hydroxypropionic acid in the presence of acalcium or maagnesium salt may comprise fermenting a bacterium strain having 3-hydroxypropionic acid-producing ability in the presence of a carbon source to produce a 3-hydroxypropionic acid fermentation liquor; and concentrating the fermentation liquid to form the concentrate containing 100 g/L or more of the 3-hydroxypropionic acid.

The bacterium strain having 3-hydroxypropionic acid-producing ability may include genes encoding at least one or two proteins selected from the group consisting of glycerol dehydratase and aldehyde dehydrogenase.

In one example, the 3-hydroxypropionic acid-producing strain may further include a gene (gdrAB) encoding glycerol dehydratase reactivator (GdrAB). In one example, the 3-hydroxypropionic acid-producing strain may be a bacterium strain that is further capable of biosynthesizing vitamin B12.

The glycerol dehydratase may be encoded by dhaB (GenBank accession no. U30903.1) gene, but is not limited thereto. The dhaB gene may be an enzyme derived from *Klebsiella pneumonia,* but is not limited thereto. The gene encoding the glycerol dehydratase may include a gene encoding dhaB1, dhaB2 and/or dhaB3. The glycerol dehydratase protein and the gene encoding it may include mutations in the gene and/or amino acid sequence within the range of maintaining an enzyme activity that decomposes glycerol to 3-hydroxypropanal (3-HPA) and water ($H_2O$).

The gene (aldH) encoding the aldehyde dehydrogenase (ALDH) may be, for example, aldH (GenBank Accession no. U00096.3; EaldH) gene derived from *Escherichia coli* or *E. coli* K12 MG1655 cell line, puuC gene derived from *Klebsiella pneumoniae,* and/or KGSADH gene derived from *Azospirillum brasilense,* but is not limited thereto. The aldehyde dehydrogenase protein and the gene encoding it may include mutations in the gene and/or amino acid sequence within the range of maintaining the activity for producing 3-hydroxypropionic acid from 3-hydroxypropanal.

The medium for producing the fermentation liquid can be selected without limitation within the range for the purposes for producing 3-hydroxypropionic acid. In one example, the medium may contain glycerol as a carbon source. In another example, the medium may be crude glycerol and/or pretreated crude glycerol, but is not limited thereto. In one example, the production medium may further include vitamin B12.

In the step of fermenting a bacterium strain having 3-hydroxypropionic acid-producing ability in the presence of a carbon source to produce a 3-hydroxypropionic acid fermentation liquor, the concentration of 3-hydroxypropionic acid contained in the 3-hydroxypropionic acid fermentation liquid may be 1 to 200 g/L, 10 to 150 g/L, 30 to 130 g/L, or 40 to 100 g/L.

Further, the fermentation may be a neutral fermentation, and for example, it may be maintained in the pH range of 6 to 8, 6.5 to 8, 6 to 7.5, or 6.5 to 7.5 during fermentation, but is not limited thereto. The pH range can be appropriately adjusted as needed. The calcium or magnesium salt may be added for the neutral fermentation. Further, the calcium or magnesium salt may be $Ca(OH)_2$ or $Mg(OH)_2$ , but is not limited thereto.

The method for preparing a 3-hydroxypropionate crystal may further include removing (separating) cells from the fermentation liquid; purifying and/or decolorizing the fermentation liquid and/or the fermentation liquid from which cells has been removed; and/or filtering the fermentation liquid and/or the fermentation liquid from which cells have been removed, after the step of producing the 3-hydroxypropionic acid fermentation liquid.

Removal (separation) of the cells may be carried out by selecting methods known in the art without limitation within the range of the purpose of cell (strain) removal. In one example, the separation of the cells may be carried out by centrifugation.

The step of purifying and/or decolorizing the fermentation liquid and/or the fermentation liquid from which cells have been removed can be carried out by selecting methods known in the art without limitation within the range of the purpose of purification of the fermentation liquid. For example, the step can be carried out by mixing activated carbon with the fermentation liquid and then removing the activated carbon, but is not limited thereto.

The step of filtering the fermentation liquid and/or the fermentation liquid from which cells have been removed can be carried out by selecting methods known in the art without limitation within the range of the purposes of removal of solid impurities, removal of proteins and/or materials with hydrophobic functional groups, and/or decoloration. For example, the step can be carried out by filter filtration and/or activated carbon filtration methods, but is not limited thereto.

The method for preparing a 3-hydroxypropionate crystal may comprise concentrating the fermentation liquid to form the concentrate containing 100 g/L or more of 3-hydroxypropionic acid, after the step of fermenting the bacterium strain having 3-hydroxypropionic acid-producing ability to produce a 3-hydroxypropionic acid fermentation liquid.

Concentration of the fermentation liquid may be carried out by evaporating the fermentation liquid (e.g., a liquid component of the fermentation liquid).

The concentration may be carried out by any means commonly available for evaporating the liquid component of the fermentation liquid. For example, the concentration may be carried out by rotary evaporation, evaporation concentration, vacuum concentration, reduced pressure concentration, and the like, but is not limited thereto.

In one example, the concentration of 3-hydroxypropionic acid in the fermentation liquid after concentration may be increased by 2 to 50 times, 2 to 40 times, 2 to 30 times, 2 to 20 times, 2 to 10 times, 5 to 50 times, 5 to 40 times, 5 to 30 times, 5 to 20 times, or 5 to 10 times compared to before concentration.

Meanwhile, according to yet another embodiment of the invention, there can be provided a process of recovering 3-hydroxypropionic acid, comprising separating the 3-hydroxypropionate crystal obtained in the method for preparing the 3-hydroxypropionate crystal, and converting the separated crystal to 3-hydroxypropionic acid.

The method of separating the 3-hydroxypropionate crystal obtained in the method for preparing the 3-hydroxypropionate crystal can be carried out, without limitation, by selecting a method known in the technical field to which the present invention belongs within the range of the purpose of separating crystals. In one example, the recovery of the 3-hydroxypropionate crystal may be carried out by drying (e.g., drying by heating) and/or filtration, but is not limited thereto.

Further, the method of converting (purifying) the separated 3-hydroxypropionate crystal to 3-hydroxypropionic acid may be carried out by selecting a method known in the art without limitation within the range of the purpose of purifying 3-hydroxypropionic acid. For example, one or more of the methods listed below may be used, but are not limited thereto.

For example, there may be mentioned:

i) a method of removing cations through a cation exchange resin to protonate 3-hydroxypropionic acid;
ii) a method of extracting cations (e.g., Mg, Ca, etc.) with an organic solvent using a liquid cation exchange to protonate 3-hydroxypropionic acid; and
iii) a method of titrating with an acid (e.g., sulfuric acid, etc.) to produce a salt (e.g., $CaSO_4(s)$ or $MgSO_4(s)$), thereby protonating 3-hydroxypropionic acid.

Specifically, a cation exchange resin column can also be used in the process of recovering 3-hydroxypropionic acid.

Specifically, the method may comprise contacting the formed solution containing 3-hydroxypropionic acid with a cation exchange resin column; and recovering 3-hydroxypropionic acid from a solution contacted with the cation exchange resin column.

The cation exchange resin column serves to convert 3-hydroxypropionate present in a solution containing 3-hydroxypropionic acid to 3-hydroxypropionic acid, whereby 3-hydroxypropionic acid having higher purity can be separated and recovered with high efficiency.

Meanwhile, the cation exchange resin may be a polymer containing a functional group capable of ion exchange in its matrix, wherein the ion-exchangeable functional groups introduced into the matrix can be in the acid form capable of exchanging hydrogen cation ($H^+$), such as -COOH, -SOOH, and -POOH.

Further, polystyrene, acrylic polymer or the like can be used as the matrix of the cation exchange resin, and various matrixes such as homopolymers, co-polymers, block polymers and random copolymers can be used.

The particles of the cation exchange resin may have a spherical or irregular shape, and the particle size distribution range of the cation exchange resin may be 0.3 mm to 1.2 mm.

The exchange efficiency of the cation exchange resin may be 1.2 eq to 2.0 eq, or 1.6 eq to 1.9 eq, taking into account the stability of the cation exchange resin and the degree of ion desorption within the cation exchange resin.

In addition, the cation exchange resin may be used alone or in combination of two or more types, and the exchange efficiency varies depending on the type of the resin used, and thus, the resin can be selected in consideration of the particle size, shape, exchanger, and the like.

Meanwhile, the resin volume of the cation exchange resin column may be 10 BV or more, 11 BV or more, or 12 BV or more. The resin volume (BED VOLUME; BV) means the volume occupied by the filler in the column. If the resin volume content of the cation exchange resin column is too low, exchange efficiency decreases during ion exchange, and if the resin volume content is too high, the ion exchange time increases, which may be uneconomical.

## ADVANTAGEOUS EFFECTS

According to the present invention, there can be provided a 3-hydroxypropionate crystal that not only contains very low amounts of impurities, but also is easy to purify and has excellent thermal stability and shape stability, a method for preparing a high-purity 3-hydroxypropionate crystal that is easy to purify and has excellent thermal and shape stability, and a process of recovering 3-hydroxypropionic acid from the 3-hydroxypropionate crystal provided above.

Since the 3-hydroxypropionate crystal provided above contains 3-hydroxypropionate in high purity and has a specific particle size or shape, it is easy to filter from impurities such as fermentation by-products and/or additives. In addition, in the process of recovering (separating, purifying) 3-hydroxypropionic acid, by-products and/or additives can be easily separated through the crystallization of 3-hydroxypropionate, and high-purity 3-hydroxypropionate can be recovered, the process can be performed without an organic solvent, the process of separating and purifying 3-hydroxypropionic acid can be simplified, and the purification cost of 3-hydroxypropionic acid can be reduced.

## BRIEF DESCRIPTION OF THE DRAWINGS

FIG. 1 is a graph showing the results of X-ray diffraction (XRD) analysis of 3-hydroxypropionate crystals of Example 1.
FIG. 2 is a graph showing the results of X-ray diffraction (XRD) analysis of 3-hydroxypropionate crystals of Example 2.
FIG. 3 is a graph showing the results of X-ray diffraction (XRD) analysis of 3-hydroxypropionate crystals of Example 3.
FIG. 4 is a graph showing the results of X-ray diffraction (XRD) analysis of 3-hydroxypropionate crystals of Example 4.
FIG. 5 is a graph showing the results of X-ray diffraction (XRD) analysis of 3-hydroxypropionate crystals of Example 5.
FIG. 6 is a graph showing the results of X-ray diffraction (XRD) analysis of 3-hydroxypropionate crystals of Example 6.
FIG. 7 is a graph showing the results of X-ray diffraction (XRD) analysis of 3-hydroxypropionate crystals of Example 7.

## DETAILED DESCRIPTION OF THE EMBODIMENTS

Hereinafter, the present invention will be described in more detail with reference to examples. However, the following examples are for illustrative purposes only, and are not intended to limit the scope of the present invention.
All temperatures are given in degrees Celsius unless otherwise specified herein.

**Preparation Example 1: Preparation of bacterium strain for producing 3-hydroxypropionic acid**

Recombinant vectors into which genes encoding glycerol dehydratase and aldehyde dehydrogenase, which are known to produce 3-hydroxypropionic acid (3HP) using glycerol as a substrate, were introduced were manufactured. The prepared recombinant vector was introduced into *E.coli* W3110 strain to prepare a 3-hydroxypropionic acid-producing strain.
More specifically, a BtuR gene encoding adenosyltransferase was cloned into plasmid pCDF containing a gene (dhaB) encoding glycerol dehydratase, a gene (aldH) encoding aldehyde dehydrogenase and a gene (gdrAB) encoding glycerol dehydratase reactivase. The resulting pCDF_J23101_dhaB_gdrAB_J23100_aldH_btuR vector was introduced into strain W3110 (KCCM 40219) by an electroporation method using an electroporation device (Bio-Rad, Gene Pulser Xcell) to prepare 3-hydroxypropionic acid-producing strain. The process of preparing the 3-hydroxypropionic acid-producing strain of Preparation Example 1 and the vectors, primers, and enzymes used were carried out with reference to Example 1 of Korean Unexamined Patent Publication No. 10-2020-0051375,

**Example 1: Preparation of Mg(3HP)$_2$ crystal and recovery of 3-hydroxypropionic acid**

(1) Preparation of Mg(3HP)$_2$ crystal

The 3-hydroxypropionic acid-producing strain prepared in Preparation Example 1 was fermented and cultured at 35 degrees Celsius in a 5L fermenter using unpurified glycerol as a carbon source to produce 3-hydroxypropionic acid. In order to prevent the lowering of pH due to the production of 3-hydroxypropionic acid, magnesium hydroxide (Mg(OH)$_2$)was added thereto to maintain the pH to be neutral during the fermentation.
After fermentation culture, cells were removed by centrifugation (4000 rpm, 10 minutes, 4 degrees Celsius), and primary fermentation liquid purification (primary purification) was performed using activated carbon. Specifically, activated carbon was added to the fermentation liquid from which bacterial cells were removed by centrifugation, the mixture was well mixed, and then centrifuged again to separate the activated carbon. Then, the fermentation liquid from which the activated carbon was separated was filtered with a vacuum pump through a 0.7 um filter paper to purify the 3-hydroxypropionic acid fermentation liquid.
The concentration of 3-hydroxypropionic acid in the fermentation liquid after completion of the primary purification was a level of about 100 g/L, and the fermentation liquid was concentrated to a concentration of 600 g/L using a rotary evaporator (50 degrees Celsius, 50 mbar) to prepare 2L of a concentrate, and ethanol was added in an amount of two times the volume of the concentrate, and stirred (80 rpm) at room temperature to produce Mg(3HP)$_2$ crystals. At this time, the concentration of the magnesium salt in the concentrate was 493.3 g/L (based on Mg(OH)$_2$).
The resulting crystals were washed three times with ethanol (EtOH) and dried in an oven at 50 degrees Celsius to finally recover the crystals.

(2) Recovery of 3-hydroxypropionic acid

A cation exchange resin column (TRILITE®CMP28LH, Samyang Corporation) was filled with 12 BV, and then washed with water to prepare a cation exchange resin column.

An aqueous solution containing the magnesium salt crystal of 3-hydroxypropionic acid was charged into a cation exchange resin column at a space velocity (SV) of 2.4 [$m^3h^{-1}$], and then distilled water was charged into to the cation exchange resin column at a space velocity (SV) of 7.2 [$m^3h^{-1}$] or less to obtain a 3-hydroxypropionic acid aqueous solution with a pH of 2.5 or less.

Subsequently, water was removed from the obtained 3-hydroxypropionic acid aqueous solution, and concentrated so that the concentration of 3-hydroxypropionic acid in the aqueous solution was 30 wt.%.

**Example 2: Preparation of Mg(3HP)$_2$ crystal and recovery of 3-hydroxypropionic acid**

(1) Preparation of Mg(3HP)$_2$ crystal

Mg(3HP)$_2$ crystals were prepared in the same manner as in Example 1, except that ethanol was added in an amount of 1 time the volume of the concentrate, and stirred (300 rpm) at room temperature to produce Mg (3HP)$_2$ crystals.

(2) Recovery of 3-hydroxypropionic acid

A 30 wt.% 3-hydroxypropionic acid aqueous solution was obtained in the same manner as in Example 1, except that the aqueous solution containing the magnesium salt crystal of 3-hydroxypropionic acid obtained above was used.

**Example 3: Preparation of Mg(3HP)$_2$ crystal and recovery of 3-hydroxypropionic acid**

(1) Preparation of Mg(3HP)$_2$ crystal

Mg(3HP)$_2$ crystal was prepared in the same manner as in Example 1, except that the fermentation liquid was concentrated to a concentration of 600 g/L using a rotary evaporator (50 degrees Celsius, 50 mbar) to prepare 2000 g/L of a concentrate, and ethanol was added in an amount of 2 times the volume of the concentrate, and stirred (120 rpm) at room temperature to produce Mg(3HP)$_2$ crystal.

(2) Recovery of 3-hydroxypropionic acid

A 30 wt.% 3-hydroxypropionic acid aqueous solution was obtained in the same manner as in Example 1, except that the aqueous solution containing the magnesium salt crystal of 3-hydroxypropionic acid obtained above was used,

**Example 4: Preparation of Ca(3HP)$_2$ crystal and recovery of 3-hydroxypropionic acid**

(1) Preparation of Ca(3HP)$_2$ crystal

Ca(3HP)$_2$ crystal was prepared in the same manner as in Example 1, except that calcium hydroxide (Ca(OH)$_2$) was used instead of magnesium hydroxide (Mg(OH)$_2$).

(2) Recovery of 3-hydroxypropionic acid

A 30 wt.% 3-hydroxypropionic acid aqueous solution was obtained in the same manner as in Example 1, except that the aqueous solution containing the calcium salt crystal of 3-hydroxypropionic acid obtained above was used.

**Example 5: Preparation of Ca(3HP)$_2$ crystal and recovery of 3-hydroxypropionic acid**

(1) Preparation of Ca(3HP)$_2$ crystal

Ca(3HP)$_2$ crystal was prepared in the same manner as in Example 2, except that calcium hydroxide (Ca(OH)$_2$) was used instead of magnesium hydroxide (Mg(OH)$_2$).

(2) Recovery of 3-hydroxypropionic acid

A 30 wt.% 3-hydroxypropionic acid aqueous solution was obtained in the same manner as in Example 2, except that the aqueous solution containing the calcium salt crystal of 3-hydroxypropionic acid obtained above was used.

## Example 6: Preparation of Ca(3HP)$_2$ crystal and recovery of 3-hydroxypropionic acid

(1) Preparation of Ca(3HP)$_2$ crystal

Ca(3HP)$_2$ crystal was prepared in the same manner as in Example 3, except that calcium hydroxide (Ca(OH)$_2$) was used instead of magnesium hydroxide (Mg(OH)$_2$).

(2) Recovery of 3-hydroxypropionic acid

A 30 wt.% 3-hydroxypropionic acid aqueous solution was obtained in the same manner as in Example 3, except that the aqueous solution containing the calcium salt crystal of 3-hydroxypropionic acid obtained above was used.

## Example 7: Preparation of Ca(3HP)$_2$ crystal and recovery of 3-hydroxypropionic acid

(1) Preparation of Ca(3HP)$_2$ crystal

Ca(3HP)$_2$ crystal was prepared in the same manner as in Example 6, except that ethanol was added in an amount of 2 times the volume of the concentrate

(2) Recovery of 3-hydroxypropionic acid

A 30 wt.% 3-hydroxypropionic acid aqueous solution was obtained in the same manner as in Example 3, except that the aqueous solution containing the calcium salt crystal of 3-hydroxypropionic acid obtained above was used.

## Comparative Example 1: Recovery of 3-hydroxypropionic acid

After the fermentation process was carried out in the same manner as in Example 1, the fermentation liquid after completion of the primary purification was titrated up to pH 2 using sulfuric acid (H$_2$SO$_4$). The precipitate formed during the titration process was removed to further protonate 3-hydroxypropionic acid.

The fermentation liquid from which the precipitate was removed was primarily purified and concentrated in substantially the same manner as in Example 1, and whether or not crystals were formed was confirmed. However, no crystals were formed when concentration was performed at 600 g/L (based on 3HP) during the concentration process, and the formation of crystals was not confirmed even when concentration was performed up to 1000 g/L (based on 3HP).

The cation exchange resin [TRILITE®CMP28LH, Samyang Corporation] was filled with 12 BV, and washed with water to prepare a cation exchange resin column. The obtained concentrate was charged into a cation exchange resin column at a space velocity (SV) of 2.4 [m$^3$h$^{-1}$], and then distilled water was charged into the cation exchange resin column at a space velocity (SV) of 7.2 [m$^3$h$^{-1}$] or less to obtain a 3-hydroxypropionic acid aqueous solution with a pH of 2.5 or less.

Subsequently, water was removed from the obtained 3-hydroxypropionic acid aqueous solution, and concentrated so that the concentration of 3-hydroxypropionic acid in the aqueous solution was 30 wt.%.

## Comparative Example 2: Recovery of 3-hydroxypropionic acid

After the fermentation process was carried out in the same manner as in Example 4, the fermentation liquid after completion of the primary purification was titrated up to pH 2 using sulfuric acid (H$_2$SO$_4$). The precipitate generated during the titration process was removed to further protonate 3-hydroxypropionic acid.

The fermentation liquid from which the precipitate was removed was primarily purified and concentrated in substantially the same manner as in Example 4, and whether or not crystals were formed was confirmed. However, no crystals were formed when concentration was performed at 600 g/L (based on 3HP) during the concentration process, and the formation of crystals was not confirmed even when concentration was performed up to 1000 g/L (based on 3HP).

The cation exchange resin [TRILITE®CMP28LH, Samyang Corporation] was filled with 12 BV, and washed with water to prepare a cation exchange resin column. The obtained concentrate was charged into a cation exchange resin column at a space velocity (SV) of 2.4 [m$^3$h$^{-1}$], and then distilled water was charged into the cation exchange resin column at a space velocity (SV) of 7.2 [m$^3$h$^{-1}$] or less to obtain a 3-hydroxypropionic acid aqueous solution with a pH of 2.5 or less.

Subsequently, water was removed from the obtained 3-hydroxypropionic acid aqueous solution, and concentrated so that the concentration of 3-hydroxypropionic acid in the aqueous solution was 30 wt.%.

**<Test Example>**

**1. X-ray diffraction (XRD) analysis for 3-hydroxypropionate crystals**

The crystals prepared in Examples were irradiated with Cu-K$\alpha$ rays having a wavelength of 1.54 Å, and X-ray diffraction (XRD) patterns in a reflection mode were measured.

A Bruker AXS D4 Endeavor XRD was used as the measurement device. The voltage and current used were 40 kV and 40 mA, respectively, and the optics and detectors used are as follows.

- Primary (incident beam) optics: motorized divergence slit, soller slit 2.3°
- Secondary (diffracted beam) optics: soller slit 2.3°
- LynxEye detector (1D detector)

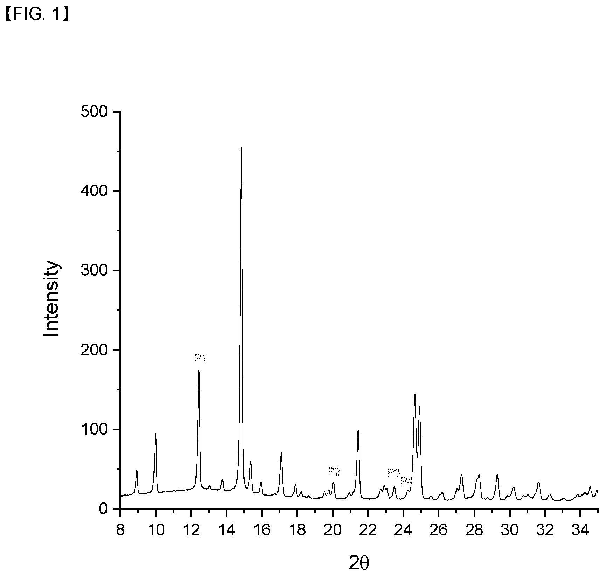

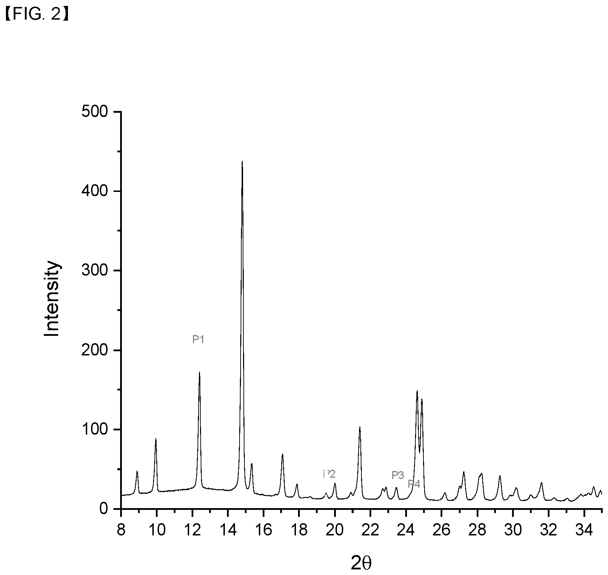

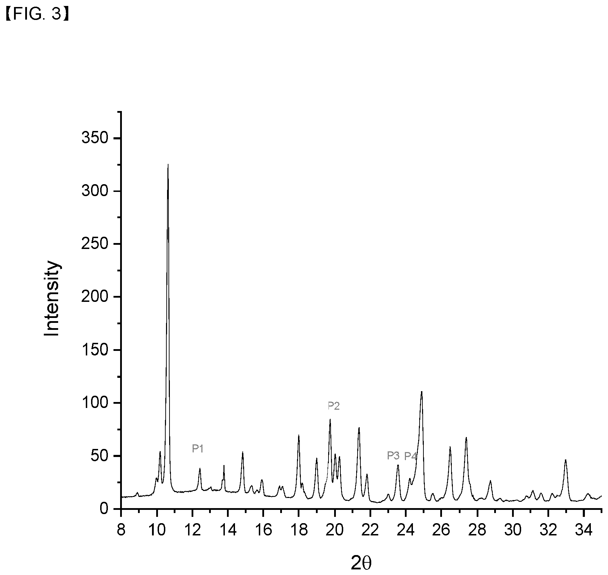

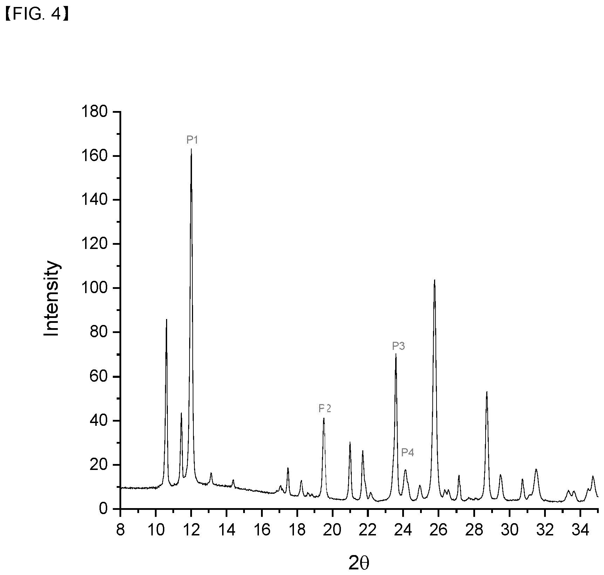

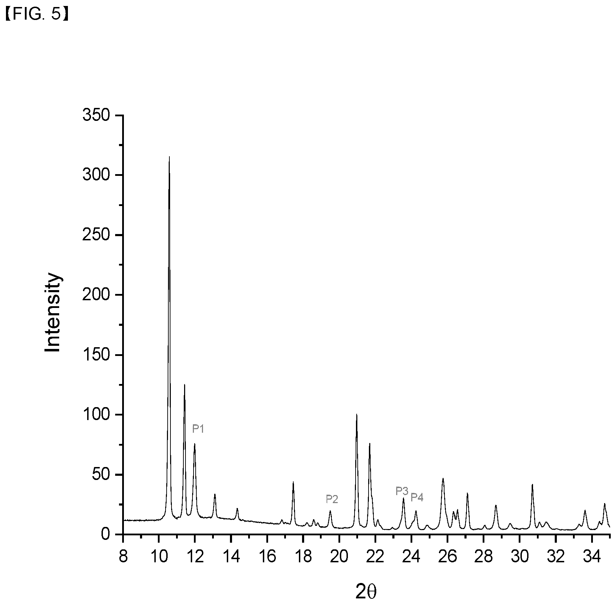

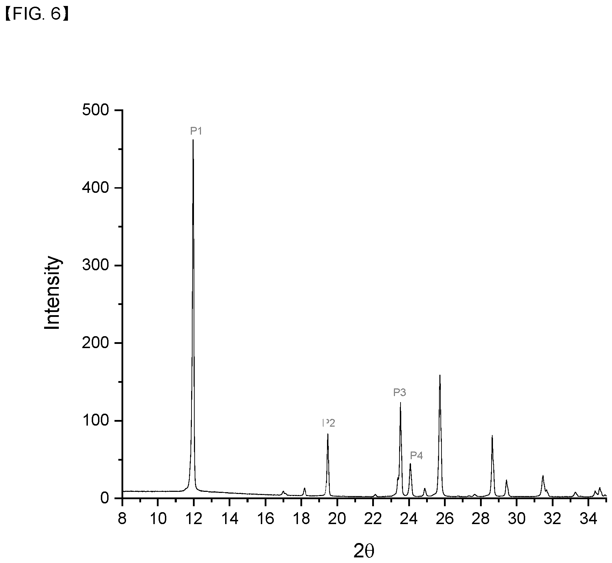

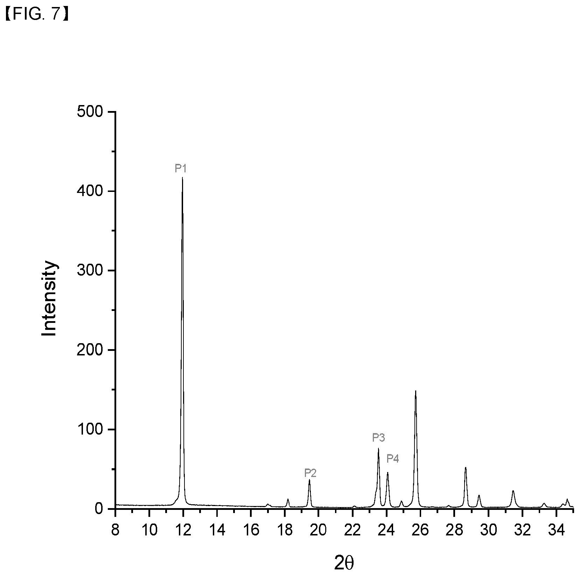

FIGS. 1 to 7 are graphs showing the results of X-ray diffraction analysis of 3-hydroxypropionate crystals of Examples 1 to 7.

**2. Particle size analysis of 3-hydroxypropionate crystals**

$D_{10}$, $D_{50}$, $D_{90}$, volume average particle size, number average particle size, and volume average particle size of the 3-hydroxypropionate crystals of Examples 1 to 7 were respectively measured using a size and shape particle analyzer (Microtrac TurboSync), and the results are shown in Table 2 below.

**3. Measurement of impurity content**

Impurities contained in the solutions finally recovered in Examples and Comparative Examples were measured by a nuclear magnetic resonance spectroscopy (NMR) and a high performance liquid chromatography (HPLC).

(1) High-Performance Liquid Chromatography (HPLC)

The ratio of impurities (glycerol, acetate and 1,3-propanediol) and 3-hydroxypropionic acid (3HP) was measured using HPLC.

For HPLC, 0.5 mM sulfuric acid ($H_2SO_4$) was flowed at a flow rate of 0.4 mL/min using an Aminex HPX-87H ion exclusion column (7.8 * 300 mm, 9 $\mu$m), the temperature of the column was 35°C, the injection volume was 5 $\mu$L, and the UV detector was set to 210 nm.

(2) $^1$H-NMR

The content ratio of impurities (glycerol, acetate, and 1,3-propanediol) and 3-hydroxypropionic acid (3HP) was analyzed using $^1$H-NMR.

100 $\mu$L of unpurified calcium or magnesium 3-hydroxypropionate and purified 3-hydroxypropionic acid were sampled, and dissolved in 500 $\mu$L of D2O which is NMR solvent, and a solution added with 11.5 $\mu$L of dimethylformamide (DMF) where moisture was removed was transferred to an NMR tube to prepare a sample for $^1$H-NMR measurement.

$^1$H-NMR spectrum (AVANCE III HD FT-NMR spectrometer (500 MHz for 1H), manufactured by Bruker) was measured using the sample for measurement.

[Table 1]

| | | XRD | | | | |
|---|---|---|---|---|---|---|
| | | Peak | 2θ value(°) | Molecular spacing (Å) | Full width at half maximum (Å) | Gran domain size (nm) |
| Example 1 | | #1 | 12.45 | 7.10 | 0.0023 | 60.36 |
| | | #2 | 20.93 | 4.24 | 0.0022 | 64.06 |
| | | #3 | 23.48 | 3.78 | 0.0028 | 50.56 |
| | | #4 | 24.66 | 3.61 | 0.0011 | 129.0 |
| Example 2 | | #1 | 12.41 | 7.12 | 0.0024 | 58.09 |
| | | #2 | 20.89 | 4.25 | 0.0027 | 52.20 |
| | | #3 | 23.45 | 3.79 | 0.0029 | 48.81 |
| | | #4 | 24.62 | 3.61 | - | - |
| Example 3 | | #1 | 12.43 | 7.11 | 0.0023 | 60.62 |
| | | #2 | 19.73 | 4.49 | 0.0018 | 78.16 |
| | | #3 | 23.56 | 3.77 | 0.0035 | 56.06 |
| | | #4 | 24.22 | 3.67 | 0.0023 | 67.13 |
| Example 4 | | #1 | 12 | 7.37 | 0.0027 | 51.62 |
| | | #2 | 19.53 | 4.54 | 0.0026 | 54.09 |
| | | #3 | 23.58 | 3.77 | 0.0029 | 48.82 |
| | | #4 | 24.12 | 3.69 | 0.0045 | 31.50 |
| Example 5 | | #1 | 11.97 | 7.38 | 0.0026 | 53.60 |
| | | #2 | 19.49 | 4.55 | 0.0026 | 54.09 |
| | | #3 | 23.56 | 3.77 | 0.0029 | 48.82 |
| | | #4 | 24.24 | 3.67 | 0.0038 | 37.31 |
| Example 6 | | #1 | 11.97 | 7.38 | 0.0018 | 77.42 |
| | | #2 | 19.47 | 4.55 | 0.0018 | 78.12 |
| | | #3 | 23.52 | 3.78 | 0.0021 | 67.42 |
| | | #4 | 24.06 | 3.69 | 0.0022 | 64.41 |
| Example 7 | | #1 | 11.97 | 7.38 | 0.0022 | 63.34 |
| | | #2 | 19.47 | 4.55 | 0.0022 | 63.92 |
| | | #3 | 23.52 | 3.78 | 0.0027 | 55.98 |
| | | #4 | 24.06 | 3.69 | 0.0027 | 52.49 |
| Comparative Example 1 | | - | - | - | - | - |
| Comparative Example 2 | | - | - | - | - | - |

[Table 2]

| | Particle size distribution | | | | Impurity content (ppm) | | |
|---|---|---|---|---|---|---|---|
| | $D_{10}$ (μm) | $D_{50}$ (μm) | $D_{90}$ (μm) | $(D_{90}-D_{10})/D_{50}$ | Glycerol | 1,3-propandiol | Acetate |
| Example 1 | 4.1 | 10 | 22.6 | 1.85 | Detection limit | Detection limit | Detection limit |
| Example 2 | 5.8 | 11.1 | 19.9 | 1.27 | Detection limit | Detection limit | Detection limit |

(continued)

| | Particle size distribution | | | | Impurity content (ppm) | | |
|---|---|---|---|---|---|---|---|
| | $D_{10}$ ($\mu$m) | $D_{50}$ ($\mu$m) | $D_{90}$ ($\mu$m) | $(D_{90}-D_{10})/D_{50}$ | Glycerol | 1,3-propandiol | Acetate |
| Example 3 | 16.1 | 56.3 | 175 | 2.82 | Detection limit | Detection limit | Detection limit |
| Example 4 | 44.5 | 85 | 146.5 | 1.2 | Detection limit | Detection limit | Detection limit |
| Example 5 | 24.2 | 70.2 | 158.6 | 1.91 | Detection limit | Detection limit | Detection limit |
| Example 6 | 75.6 | 158 | 201 | 0.79 | Detection limit | Detection limit | Detection limit |
| Example 7 | 74.1 | 140 | 196 | 0.87 | Detection limit | Detection limit | Detection limit |
| Comparative Example 1 | | | | | 3126 | Detection limit | 200 |
| Comparative Example 2 | | | | | 2425 | Detection limit | Detection limit |

Referring to Tables 1 and 2, it was confirmed that 3-hydroxypropionate crystals obtained in Examples 1 to 7 show one peak at a diffraction angle ($2\theta\pm0.2°$) of 11.97 to 12.45 in an X-ray diffraction spectrum (XRD), the full width at half maximum (Å) of the first peak is 0.0018 to 0.0027, and the gran domain size (nm) of the first peak is 51.62 to 77.42. Also, it was confirmed that the particle size distribution $D_{50}$ of Examples was 10 to 158 $\mu$m, and $(D_{90}-D_{10})/D_{50}$ was 0.79 to 2.82.

In addition, it was confirmed that the obtained 3-hydroxypropionate crystal exhibited a second peak at the diffraction angle ($2\theta\pm0.2°$) of 19.47 to 20.93 in the X-ray diffraction spectrum (XRD), and exhibited a third peak at a diffraction angle ($2\theta\pm0.2°$) of 23.45 to 23.58 and a fourth peak at a diffraction angle ($2\theta\pm0.2°$) of 24.06 to 24.66.

Since the 3-hydroxypropionate crystal provided above contains 3-hydroxypropionate in high purity and has a specific particle size or shape, it is easy to filter from impurities such as fermentation by-products and/or additives. In addition, in the process of recovering (separating, purifying) 3-hydroxypropionic acid, by-products and/or additives can be easily separated through the crystallization of 3-hydroxypropionate, and high-purity 3-hydroxypropionate can be recovered, the process can be performed without an organic solvent, the process of separating and purifying 3-hydroxypropionic acid can be simplified, and the purification cost of 3-hydroxypropionic acid can be reduced.

## 4. Analysis of biocarbon content in 3-hydroxypropionate crystals

The radioactive carbon isotope ratio (pMC) and biocarbon content of 3-hydroxypropionate crystals (Mg(3HP)$_2$) of Example 1 and 3-hydroxypropionate crystals (Ca(3HP)$_2$) of Example 4 were analyzed using ASTM D 6866-21 (Method B), and the results are shown in Table 3 below.

The biocarbon content refers to the content of biocarbon contained in the 3-hydroxypropionate crystals of Examples 1 and 4, respectively, and the radioactive carbon isotope ratio (pMC) refers to the ratio of the radioactive carbon isotope ($^{14}$C) contained in the 3-hydroxypropionate crystal to the radioactive carbon isotope ($^{14}$C) of the modern standard reference material.

[Table 3]

| | Concentrated concentration (g/L) | Radioactive carbon isotope ratio (pMC) | Biocarbon content (%) |
|---|---|---|---|
| Example 1 | 600 | 101.52 | 100 |
| Example 4 | 600 | 102.23 | 100 |
| - pMC: percent Modern Carbon | | | |

Referring to Table 3, it was confirmed that all of the 3HP salt crystals of Examples 1 and 4 had a pMC of 101 or more and a biocarbon content of 100%.

**Claims**

1. A 3-hydroxypropionate crystal which:

   comprises a calcium salt of 3-hydroxypropionic acid or a hydrate thereof; or a magnesium salt of 3-hydroxypropionic acid or a hydrate thereof,
   comprises a first peak at a diffraction angle ($2\theta \pm 0.2°$) of 11.0 to 13.0 in an X-ray diffraction spectrum (XRD).

2. The 3-hydroxypropionate crystal according to claim 1, which:

   comprises a second peak at a diffraction angle ($2\theta \pm 0.2°$) of 19.0 to 21.0 in an X-ray diffraction spectrum (XRD), or
   comprises a third peak at a diffraction angle ($2\theta \pm 0.2°$) of 22.0 to 23.80 in an X-ray diffraction spectrum (XRD), or
   comprises a fourth peak at a diffraction angle ($2\theta \pm 0.2°$) of 24.0 to 25.00 in an X-ray diffraction spectrum (XRD).

3. The 3-hydroxypropionate crystal according to claim 1, wherein:
   a grain domain size of 3-hydroxypropionate calculated from the full width at half maximum in the first peak is 30 to 100 nm.

4. The 3-hydroxypropionate crystal according to claim 1, wherein:

   the 3-hydroxypropionate crystal has a particle size distribution $D_{50}$ of 5 $\mu$m or more and 180 $\mu$m or less, and $(D_{90}\text{-}D_{10})/D_{50}$ of the 3-hydroxypropionate crystal is 0.70 or more and 3.00 or less, and
   particle size distributions $D_{50}$, $D_{10}$ and $D_{90}$ mean particle sizes at which cumulative volumes of particles reach 50%, 10% and 90%, respectively, in the particle size distribution curve.

5. The 3-hydroxypropionate crystal according to claim 1, wherein:
   the 3-hydroxypropionate crystal has a radiocarbon isotope content of 20 pMC (percent modern carbon) or more, and a biocarbon content of 20 wt.% or more, as measured according to the standard of ASTM D6866-21.

6. The 3-hydroxypropionate crystal according to claim 1, wherein:

   a content of residual components containing glycerol, 1,3-propanediol, and acetate is less than 100 ppmw, and
   the 3-hydroxypropionate crystal has a purity of 70% or more.

7. A method for preparing a 3-hydroxypropionate crystal, comprising:

   forming a concentrate containing 100 g/L or more of 3-hydroxypropionic acid in the presence of $\underline{Ca(OH)_{2,2}}$ or a mixture thereof; and
   contacting the concentrate with a nonsolvent to form the 3-hydroxypropionate crystal.

8. The method for preparing a 3-hydroxypropionate crystal according to claim 7, wherein:
   the concentrate and the nonsolvent are used in a volume ratio of 1:0.5 to 1:20.

9. The method for preparing a 3-hydroxypropionate crystal according to claim 7, wherein:
   the nonsolvent includes an alcohol-based nonsolvent, a ketone-based nonsolvent, a nitrile-based nonsolvent, or a mixture of two or more thereof.

10. The method for preparing a 3-hydroxypropionate crystal according to claim 7, wherein:
    the contacting of the concentrate with a nonsolvent to form a 3-hydroxypropionate crystal is carried out at a temperature of 0°C to 100°C.

11. The method for preparing a 3-hydroxypropionate crystal according to claim 7, wherein:
    the concentrate contains 100 g/L or more and 1000 g/L or less of the 3-hydroxypropionic acid.

12. The method for preparing a 3-hydroxypropionate crystal according to claim 7, wherein:

    the forming of a concentrate containing 100 g/L or more of 3-hydroxypropionic acid in the presence of $Ca(OH)_2$, $Mg(OH)_2$ or a mixture thereof comprises,

17

fermenting a bacterium strain having 3-hydroxypropionic acid-producing ability in the presence of a carbon source to produce a 3-hydroxypropionic acid fermentation liquid; and
concentrating the fermentation liquid to form the concentrate containing 100 g/L or more of the 3-hydroxypropionic acid.

13. A process of recovering 3-hydroxypropionic acid, comprising separating the 3-hydroxypropionate crystal obtained in the method for preparing the 3-hydroxypropionate crystal according to claim 7, and converting the separated crystal to 3-hydroxypropionic acid.

**Patentansprüche**

1. 3-Hydroxypropionatkristall, der:

ein Calciumsalz von 3-Hydroxypropionsäure oder ein Hydrat davon; oder ein Magnesiumsalz von 3-Hydroxypropionsäure oder ein Hydrat davon umfasst,
einen ersten Peak bei einem Beugungswinkel ($2\theta\pm0,2°$) von 11,0 bis 13,0 in einem Röntgenbeugungsspektrum (XRD) umfasst.

2. 3-Hydroxypropionatkristall nach Anspruch 1, der:

einen zweiten Peak bei einem Beugungswinkel ($2\theta\pm0,2°$) von 19,0 bis 21,0 in einem Röntgenbeugungsspektrum (XRD) umfasst, oder
einen dritten Peak bei einem Beugungswinkel ($2\theta\pm0,2°$) von 22,0 bis 23,80 in einem Röntgenbeugungsspektrum (XRD) umfasst, oder
einen vierten Peak bei einem Beugungswinkel ($2\theta\pm0,2°$) von 24,0 bis 25,00 in einem Röntgenbeugungsspektrum (XRD) umfasst.

3. 3-Hydroxypropionatkristall nach Anspruch 1, wobei:
eine Korndomänengröße von 3-Hydroxypropionat, berechnet aus der Halbwertsbreite in dem ersten Peak, 30 bis 100 nm beträgt.

4. 3-Hydroxypropionatkristall nach Anspruch 1, wobei:

der 3-Hydroxypropionatkristall eine Teilchengrößenverteilung $D_{50}$ von 5 $\mu$m oder mehr und 180 $\mu$m oder weniger aufweist, und
($D_{90}$-$D_{10}$)/$D_{50}$ des 3-Hydroxypropionatkristalls 0,70 oder mehr und 3,00 oder weniger beträgt, und
Teilchengrößenverteilungen $D_{50}$, $D_{10}$ und $D_{90}$ Teilchengrößen bedeuten, bei denen kumulative Volumina von Teilchen 50 %, 10 % bzw. 90 % in der Teilchengrößenverteilungskurve erreichen.

5. 3-Hydroxypropionatkristall nach Anspruch 1, wobei:
der 3-Hydroxypropionatkristall einen Gehalt an Radiokohlenstoffisotopen von 20 pMC (Prozent moderner Kohlenstoff) oder mehr und einen Biokohlenstoffgehalt von 20 Gew.-% oder mehr, wie gemäß dem Standard von ASTM D6866-21 gemessen, aufweist.

6. 3-Hydroxypropionatkristall nach Anspruch 1, wobei:

ein Gehalt an Restkomponenten, die Glycerin, 1,3-Propandiol und Acetat enthalten, weniger als 100 ppmw beträgt und
der 3-Hydroxypropionatkristall eine Reinheit von 70 % oder mehr aufweist.

7. Verfahren zur Herstellung eines 3-Hydroxypropionatkristalls, umfassend:

Bilden eines Konzentrats, das 100 g/l oder mehr 3-Hydroxypropionsäure enthält, in Gegenwart von Ca(OH)$_2$ , Mg(OH)$_2$ oder einer Mischung davon; und
Inkontaktbringen des Konzentrats mit einem Nichtlösungsmittel, um den 3-Hydroxypropionatkristall zu bilden.

8. Verfahren zur Herstellung eines 3-Hydroxypropionatkristalls nach Anspruch 7, wobei: das Konzentrat und das Nichtlösungsmittel in einem Volumenverhältnis von 1:0,5 bis 1:20 verwendet werden.

9. Verfahren zur Herstellung eines 3-Hydroxypropionatkristalls nach Anspruch 7, wobei:
das Nichtlösungsmittel ein Nichtlösungsmittel auf Alkoholbasis, ein Nichtlösungsmittel auf Ketonbasis, ein Nichtlösungsmittel auf Nitrilbasis oder eine Mischung von zwei oder mehr davon umfasst.

10. Verfahren zur Herstellung eines 3-Hydroxypropionatkristalls nach Anspruch 7, wobei:
das Inkontaktbringen des Konzentrats mit einem Nichtlösungsmittel, um einen 3-Hydroxypropionatkristall zu bilden, bei einer Temperatur von 0 °C bis 100 °C durchgeführt wird.

11. Verfahren zur Herstellung eines 3-Hydroxypropionatkristalls nach Anspruch 7, wobei:
das Konzentrat 100 g/l oder mehr und 1000 g/l oder weniger der 3-Hydroxypropionsäure enthält.

12. Verfahren zur Herstellung eines 3-Hydroxypropionatkristalls nach Anspruch 7, wobei:
das Bilden eines Konzentrats, das 100 g/l oder mehr 3-Hydroxypropionsäure enthält, in Gegenwart von $Ca(OH)_2$ , $Mg(OH)_2$ oder einer Mischung davon umfasst:

Fermentieren eines Bakterienstamms mit 3-Hydroxypropionsäure-Produktionsfähigkeit in Gegenwart einer Kohlenstoffquelle, um eine 3-Hydroxypropionsäure-Fermentationsflüssigkeit zu produzieren; und
Konzentrieren der Fermentationsflüssigkeit, um das Konzentrat zu bilden, das 100 g/l oder mehr der 3-Hydroxypropionsäure enthält.

13. Verfahren zur Gewinnung von 3-Hydroxypropionsäure, umfassend das Abtrennen des 3-Hydroxypropionatkristalls, der in dem Verfahren zur Herstellung des 3-Hydroxypropionatkristalls nach Anspruch 7 erhalten wurde, und Umwandeln des abgetrennten Kristalls in 3-Hydroxypropionsäure.

**Revendications**

1. Cristal de 3-hydroxypropionate qui :

comprend un sel de calcium d'acide 3-hydroxypropionique ou un hydrate de celui-ci ; ou un sel de magnésium d'acide 3-hydroxypropionique ou un hydrate de celui-ci,
comprend un premier pic à un angle de diffraction $(2\theta\pm0,2°)$ de 11,0 à 13,0 dans un spectre de diffraction des rayons X (XRD).

2. Cristal de 3-hydroxypropionate selon la revendication 1 qui :

comprend un deuxième pic à un angle de diffraction $(2\theta\pm0,2°)$ de 19,0 à 21,0 dans un spectre de diffraction des rayons X (XRD), ou
comprend un troisième pic à un angle de diffraction $(2\theta\pm0,2°)$ de 22,0 à 23,80 dans un spectre de diffraction des rayons X (XRD), ou
comprend un quatrième pic à un angle de diffraction $(2\theta\pm0,2°)$ de 24,0 à 25,0 dans un spectre de diffraction des rayons X (XRD).

3. Cristal de 3-hydroxypropionate selon la revendication 1, dans lequel :
une taille de domaine de grain de 3-hydroxypropionate calculée à partir de la largeur entière à mi-hauteur dans le premier pic est de 30 à 100 nm.

4. Cristal de 3-hydroxypropionate selon la revendication 1, dans lequel :

le cristal de 3-hydroxypropionate a une distribution granulométrique $D_{50}$ de 5 $\mu$m ou plus et de 180 $\mu$m ou moins, et
le $(D_{90}-D_{10})/D_{50}$ du cristal de 3-hydroxypropionate est de 0,70 ou plus et de 3,00 ou moins, et
les distributions granulométriques $D_{50}$, $D_{10}$ et $D_{90}$ signifient des tailles de particules auxquelles les volumes cumulatifs des particules atteignent 50 %, 10 % et 90 %, respectivement, sur la courbe de distribution granulométrique.

**5.** Cristal de 3-hydroxypropionate selon la revendication 1, dans lequel :
le cristal de 3-hydroxypropionate a un contenu en isotope de radiocarbone de 20 pMC (pourcentage de carbone moderne) ou plus, et un contenu en biocarbone de 20 % en poids ou plus, tel que mesuré conformément à la norme ASTM D6866-21.

**6.** Cristal de 3-hydroxypropionate selon la revendication 1, dans lequel :

un contenu en composants résiduels contenant du glycérol, du 1,3-propanediol et un acétate est de moins de 100 ppm en poids, et
le cristal de 3-hydroxypropionate a une pureté de 70 % ou plus.

**7.** Procédé de préparation d'un cristal de 3-hydroxypropionate, comprenant :

former un concentré contenant 100 g/l ou plus d'acide 3-hydroxypropionique en présence de $Ca(OH)_{2,2}$ ou un mélange de ceux-ci ; et
mettre le concentré en contact avec un non solvant pour former le cristal de 3-hydroxypropionate.

**8.** Procédé de préparation d'un cristal de 3-hydroxypropionate selon la revendication 7, dans lequel :
le concentré et le non solvant sont utilisés selon un rapport volumique de 1:0,5 à 1:20.

**9.** Procédé de préparation d'un cristal de 3-hydroxypropionate selon la revendication 7, dans lequel :
le non solvant comprend un non solvant à base d'alcool, un non solvant à base de cétone, un non solvant à base de nitrile ou un mélange de deux ou plus de ceux-ci.

**10.** Procédé de préparation d'un cristal de 3-hydroxypropionate selon la revendication 7, dans lequel :
la mise en contact du concentré avec un non solvant pour former un cristal de 3-hydroxypropionate est effectuée à une température de 0 °C à 100 °C.

**11.** Procédé de préparation d'un cristal de 3-hydroxypropionate selon la revendication 7, dans lequel :
le concentré contient 100 g/l ou plus et 1000 g/l ou moins de l'acide 3-hydroxypropionique.

**12.** Procédé de préparation d'un cristal de 3-hydroxypropionate selon la revendication 7, dans lequel :

former un concentré contenant 100 g/l ou plus d'acide 3-hydroxypropionique en présence de $Ca(OH)_{2,2}$ ou un mélange de ceux-ci comprend,
fermenter une souche bactérienne ayant l'aptitude de produire de l'acide 3-hydroxypropionique en présence d'une source de carbone pour produire un liquide de fermentation d'acide 3-hydroxypropionique ; et
concentrer le liquide de fermentation pour former le concentré contenant 100 g/l ou plus de l'acide 3-hydroxypropionique.

**13.** Procédé de récupération d'acide 3-hydroxypropionique, comprenant séparer le cristal de 3-hydroxypropionate obtenu dans le procédé de préparation du cristal de 3-hydroxypropionate selon la revendication 7, et convertir le cristal séparé en acide 3-hydroxypropionique.

【FIG. 1】

【FIG. 2】

【FIG. 3】

【FIG. 4】

【FIG. 5】

【FIG. 6】

【FIG. 7】

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020200051375

**Non-patent literature cited in the description**

- *CHEMICAL ENGINEERING SCIENCE*, 13 February 2012, vol. 77